# EUROPÄISCHE PATENTSCHRIFT

(11) **EP 3 376 416 B1**
(45) Veröffentlichungstag und Bekanntmachung des Hinweises auf die Patenterteilung: **21.07.2021**
(21) Anmeldenummer: 17160644.5
(22) Anmeldetag: 13.03.2017
(51) Int. Cl.: G16H 20/30, G16H 20/90, A61F 5/56, G09B 15/00, G10H 3/14

(54) **VORRICHTUNG UND VERFAHREN ZUM TRAINIEREN DER MUSKULATUR UND DES BINDEGEWEBES IN MUNDHÖHLE UND RACHEN EINER PERSON**
DEVICE AND METHOD FOR TRAINING MUSCLE AND CONNECTIVE TISSUE IN THE ORAL CAVITY AND THROAT OF A PERSON
DISPOSITIF ET PROCÉDÉ D'ENTRAÎNEMENT DE LA MUSCULATURE ET DES TISSUS DANS LA CAVITÉ BUCCALE ET DU PHARYNX DE LA TRACHÉE D'UNE PERSONNE

(43) Veröffentlichungstag der Anmeldung: 19.09.2018
(73) Patentinhaber: Asate AG, 8645 Jona (CH)
(72) Erfinder: Suarez, Alexandre, 8344 Bäretswil (CH); Suarez, Aron, 8716 Schmerikon (CH)
(74) Vertreter: EGLI-EUROPEAN PATENT ATTORNEYS

(56) Entgegenhaltungen:
- WO-A1-2012/146829
- WO-A1-2017/037342
- JP-B2- 4 510 496
- US-A- 3 482 026
- US-A- 4 779 465
- US-A- 5 569 864
- US-A1- 2007 186 757
- US-A1- 2016 372 097
- astramediaTV: "Asate AG - Therapie gegen Schnarchen & Schlafapnoe", youtube, 14. Juni 2010 (2010-06-14), Seite 1 pp., XP054977400, Gefunden im Internet: URL:https://www.youtube.com/watch?v=MGBeJk 1_gO8 [gefunden am 2017-05-31]
- M. A PUHAN ET AL: "Didgeridoo playing as alternative treatment for obstructive sleep apnoea syndrome: randomised controlled trial", BMJ, Bd. 332, Nr. 7536, 4. Februar 2006 (2006-02-04), Seiten 266-270, XP055376537, ISSN: 0959-8138, DOI: 10.1136/bmj.38705.470590.55

## Beschreibung

Die Erfindung bezieht sich auf eine Vorrichtung zum Trainieren der Muskulatur und des Bindegewebes in Mundhöhle und Rachen einer Person.

Die Firma Asate AG in Schmerikon, Schweiz, hat das Konzept eingeführt, die Muskulatur und das Bindegewebe in Mundhöhle und Rachen einer Person überhaupt zum Zwecke des Vermeidens von Atemwegsstörungen zu trainieren, etwa mithilfe eines Blasinstruments. Dadurch wird es nicht mehr nötig, operative Eingriffe an Mundhöhlen- und Rachenmuskulatur vorzunehmen. Auch sind keine nächtlich eingesetzten Hilfsmittel mehr erforderlich, welche dazu dienen, während des Schlafs die Atemwege offen zu halten.

Auf dem Gebiet der Lehrmaterialien für Musikinstrumente ist es bekannt, einen Personalcomputer zu benutzen. So kann beispielsweise eine elektrische Gitarre mit einem solchen Personalcomputer gekoppelt werden, es läuft eine Begleitmusik, und zu spielende Noten werden dem Lernenden angezeigt.

Aus der WO 2017/037342 A1 ist ein Lehrsystem zum Unterstützen des Erlernens des Spiels eines Musikinstruments bekannt, beispielsweise eines Blasinstruments. Das Lehrsystem zeigt dem Spieler Noten und dergleichen an, welche zu spielen sind. Es kann ein Mikrophon umfassen, erkennt die Audiosignale sowie unterzieht diese einer Auswertung im Hinblick auf die angezeigten Noten. Aufgrund des Ergebnisses der Auswertung wird eine nachfolgende Anzeige festgelegt. Bei einer Erweiterung kann das Lehrsystem auch optische Signale erfassen. Im Beispielsfall kann mittels eines Kamerabildes kontrolliert werden, ob der Spieler einen korrekten Fingersatz verwendet oder das Instrument richtig hält.

Aus dem YouTube Video von astramedia TV: "Asate AG - Therapie gegen Schnarchen & Schlafapnoe", youtube, 14. Juni 2010 (2010-06-14), XP054977400, Gefunden im Internet: URL:https://www. youtube.com/watch?v=MGBeJk1_gO8 [gefunden am 2017-05-31] ist es bekannt, dass durch ein Training mit Blasinstrumenten Schnarchen vorgebeugt werden kann. Gleiches ist auch der Veröffentlichung von M. A PUHAN ET AL: "Digeridoo playing as alternative treatment for obductive sleep apnoea syndrome: randomised controlled trial", BMJ, Bd. 332, Nr. 7536, 4. Februar 2006 (2006-02-04), Seiten 266-270, XP055376537, ISSN: 0959-8138, DOI: 10.1136/bmj.38705.470590.55 zu entnehmen. Die US 4 779 465 A offenbart ein Blasinstrument, bei welchem die Druckausübung etwa am Mundstück gemessen wird und für eine Rückkopplung an den Spieler genutzt wird.

Die WO 2012/146829 A1 befasst sich mit Lernprogrammen unter Nutzung von Mikrophonen, welche am Rechner angekoppelt sind, oder unter Nutzung einer direkten Ankopplung eines elektrischen Instruments an einen Rechner.

In der US 2007/186757 A1 geht es um eine Lern-App zum Musizieren.

Die US 5 569 864 A offenbart eine Vorrichtung mit einem Blasinstrument, das über einen in dem Blasinstrument angebrachtem Dämpfer mit eingebautem Mikrofon mit einem Tonverarbeitungssystem verbunden ist.

Der vorliegenden Erfindung liegt die Aufgabe zugrunde, eine verbesserte Vorrichtung zum Training der Muskulatur und des Bindegewebes in Mundhöhle und Rachen einer Person bereitzustellen.

Diese Aufgabe wird gelöst durch eine Vorrichtung mit den Merkmalen des Patentanspruchs 1.

Die Vorrichtung umfasst ein Blasinstrument und eine Einrichtung zum Erfassen eines von der Person mit dem Blasinstrument erzeugten Schalles, eine Einrichtung zum Auswerten des erfassten Schalles und eine Einrichtung zum Ausgeben einer in Abhängigkeit von einem Ergebnis des Auswertens definierten Rückmeldung an die das Blasinstrument spielende Person, wobei die Einrichtung zum Erfassen ein in dem Blasinstrument angeordnetes Mikrofon und/oder einen in dem Blasinstrument angeordneten piezoelektrischen Sensor umfasst.

Gemäss der Erfindung weist das Blasinstrument zwischen fünf und zwölf Windungen in Folge hintereinander auf. Weiterhin weist die Einrichtung zum Auswerten einen Datenspeicher auf, in welchem zumindest eine Datei mit Information gespeichert ist, welche für das Spielen des Blasinstruments vorgesehene Sollvorgaben für die Kombination von Tonhöhe und Lautstärke angibt.

Gemäß der Erfindung wird zusätzlich zu einem Blasinstrument eine Einrichtung zum Erfassen eines von der Person mit dem Blasinstrument erzeugten Schalles bereitgestellt, es wird eine Einrichtung zum Auswerten des erfassten Schalles bereitgestellt, und schließlich gibt es eine Einrichtung zum Ausgeben einer in Abhängigkeit von dem Ergebnis des Auswertens definierten Rückmeldung an die das Blasinstrument spielende Person. Mit der erfindungsgemäßen Vorrichtung wird somit eine zumindest teilweise Überwachung eines Trainings ermöglicht. Die trainierende Person muss nicht selbst wissen, ob sie die Töne getroffen oder ausreichend fest geblasen hat, sondern erfährt dies mittels der Einrichtung zum Auswerten, und es wird eine Rückmeldung an die Person gegeben. So lässt sich viel präziser ein Trainingsprogramm absolvieren. Je genauer nun wiederum dem ausgeklügelten Trainingsprogramm gefolgt wird, ein desto besserer Erfolg kann beim Training der Muskulatur und des Bindegewebes erzielt werden, insbesondere können mit höherer Wahrscheinlichkeit Atemwegs- und Schlafstörungen langfristig vermieden werden. Die Einrichtung zum Erfassen umfaßt ein in dem Blasinstrument angeordnetes Mikrofon und/oder einen in dem Blasinstrument angeordneten piezo-elektrischen Sensor. Durch die Integration der Einrichtung zum Erfassen in das Blasinstrument wird ein besonders leicht handhabbares Blasinstrument vorgesehen, und eine Kopplung mit einer gesonderten Einrichtung zum Erfassen ist nicht notwendig.

Die Einrichtung zum Auswerten weist einen Datenspeicher auf, in welchem zumindest eine Datei mit Information gespeichert ist, welche für das Spielen des Blasinstruments vorgesehene Sollvorgaben angibt. Auf diese Weise kann die Einrichtung zum Auswerten den erfassten Schall nach bestimmten Kriterium auswerten, nämlich gemäß der Sollvorgaben.

Die Sollvorgabe kann auch an die das Blasinstrument spielende Person ausgegeben werden. Zu letzterem Zweck ist bei einer weiter bevorzugten Ausführungsform vorgesehen, dass die Einrichtung zum Auswerten mit der Einrichtung zum Ausgeben gekoppelt ist und die Einrichtung zum Ausgeben auch zum Ausgeben einer Meldung an die das Blasinstrument spielende Person ausgelegt ist, mit der der Person eine Sollvorgabe angegeben wird. Die das Blasinstrument spielende Person muss also nicht von sich aus die Sollvorgabe kennen, sondern erhält beim Trainieren eine diesbezügliche Information.

Weiter ist bezüglich dem Datenspeicher bevorzugt, wenn die Information in der zumindest einen Datei zu zumindest einem Teil der Sollvorgaben jeweils auch angibt, welche Bedingung durch von der Einrichtung zum Erfassen des Schalles an die Einrichtung zum Auswerten weitergeleitete Daten erfüllt sein muss, damit diese Sollvorgabe erfüllt ist. Die Einrichtung zum Auswerten ist dann dazu ausgelegt, auf das Erfülltsein dieser Bedingung zu prüfen. Diese bevorzugte Ausführungsform basiert darauf, dass keine Sollvorgabe stets und perfekt umsetzbar ist, sondern dass ein gewisser Spielraum gegeben sein kann. Dies kann beispielsweise eine Tonhöhe des mit dem Blasinstrument erzeugten Schalles und/oder eine Lautstärke betreffen: Zu der Tonhöhe bzw. der Lautstärke ist jeweils ein Intervall angegeben, das einen Gültigkeitsbereich vorgibt. Fällt die Tonhöhe oder Lautstärke in das Intervall, gilt die Sollvorgabe als erfüllt.

Bei der Erfindung ist es vorzugsweise vorgesehen, dass die Einrichtung zum Erfassen eine mit dem Mikrofon bzw. dem piezoelektrischen Sensor gekoppelte und ferner in oder an dem Blasinstrument angeordnete Sendeeinrichtung zum Übermitteln von mithilfe des Mikrofons bzw. des piezo- elektrischen Sensors erfassten Daten an die Einrichtung zum Auswerten aufweist. Auf diese Weise wird in oder an dem Blasinstrument auch für die Ausgabe der entsprechenden Daten gesorgt, d. h. es ist keine zusätzliche Einrichtung erforderlich.

Bei der genannten Ausführungsform ist es weiter bevorzugt, wenn die Einrichtung zum Auswerten außerhalb des Blasinstruments bereitgestellt ist, sodass eine solche Einrichtung genutzt werden kann, die nicht gesondert für das Blasinstrument hergestellt werden muss. Es handelt sich hierbei beispielsweise um ein Smartphone, einen Tabletcomputer oder einen Personalcomputer. Alternativ zu dieser Ausführungsform wäre es allerdings auch möglich, die Einrichtung zum Auswerten ebenfalls in oder an dem Blasinstrument vorzusehen. Eine solche Ausführungsform hätte den Vorteil, dass man es mit einer sehr kompakten Vorrichtung zu tun hätte.

Bei der außerhalb des Blasinstruments vorgesehenen Einrichtung zum Auswerten erfolgt der Datentransfer in einer Variante über Kabel. In einer anderen Variante ist die Sendeeinrichtung dazu ausgelegt, die mithilfe des Mikrofons bzw. des piezoelektrischen Sensors erfassten Daten drahtlos an die Einrichtung zum Auswerten zu senden. Hierbei können beispielsweise herkömmliche Bluetooth® - Einrichtungen eingesetzt werden.

Damit das Blasinstrument auch gespielt werden kann, ohne ständig ein Stromkabel eingesteckt zu haben, ist vorzugsweise ein Speicher für elektrische Energie in dem Blasinstrument vorgesehen, um erstens das Mikrofon bzw. den piezoelektrischen Sensor und/oder zweitens die gegebenenfalls vorhandene Sendeeinrichtung zu betreiben.

Bei der erfindungsgemäßen Vorrichtung beinhaltet eine weitere bevorzugte Ausführungsform, dass die Einrichtung zum Auswerten des erfassten Schalles und die Einrichtung zum Ausgeben einer in Abhängigkeit vom Ergebnis des Auswertens definierten Rückmeldung an die das Blasinstrument spielende Person durch ein mobiles Datenverarbeitungsendgerät (wie etwa ein Smartphone, oder ein Tabletcomputer) bereitgestellt sind, auf dem ein entsprechendes Programm (eine App, eine "Application") gespeichert ist. Auf diese Weise kann die einem solchen Datenverarbeitungsgerät zugehörige Anzeige (Touchscreen oder dergleichen) genutzt werden, um eine Rückmeldung an den Spieler des Blasinstruments zu geben.

Bei der erfindungsgemäßen Vorrichtung ist ferner in einer Variante vorgesehen, dass das Blasinstrument zumindest abschnittsweise und vorzugsweise im tonerzeugenden Teil (etwa Rohr und Resonanzkörper) vollständig aus Holz besteht. In einer anderen Variante besteht das Blasinstrument zumindest abschnittsweise und vorzugsweise im tonerzeugenden Bereich (Rohr und Resonanzkörper) vollständig aus Kunststoff. Beide Materialien können auch miteinander kombiniert werden (z.B. Rohr aus Kunststoff und Resonanzkörper aus Holz, oder Mundstück am Rohr aus Holz und alles andere aus Kunststoff). Auf diese Weise ist das Blasinstrument kostengünstig herstellbar und kann dennoch eine gute Klangqualität aufweisen.

Ein nicht beanspruchtes Verfahren zum Trainieren der Muskulatur und des Bindegewebes in Mundhöhle und Rachen einer Personumfasst die Schritte
- Bereitstellen des Blasinstruments, ,
- Bereitstellen einer Schallaufnahmeeinrichtung in Form eines in dem Blasinstrument angeordneten Mikrofons und/oder eines in dem Blasinstrument angeordneten piezoelektrischen Sensor sowie Bereitstellen einer Datenverarbeitungseinrichtung als Auswerteeinrichtung, einer Ausgabeeinrichtung und eines Datenspeichers, in welchem zumindest eine Datei mit Information gespeichert ist, welche für das Spielen des Blasinstruments vorgesehene Sollvorgaben angibt, und wobei die Information in der zumindest einen Datei zu zumindest einem Teil der Sollvorgaben jeweils auch angibt, welche Bedingung durch von der Schallaufnahmeeinrichtung an die Auswerteinrichtung weitergeleitete Daten erfüllt sein muss, damit diese Sollvorgabe erfüllt ist,
- Ausgeben einer Sollvorgabe durch die Ausgabeeinrichtung zur Wahrnehmung an die Person,
- Empfangen von Schallsignalen durch die Schallaufnahmeeinrichtung und Weiterleiten entsprechender Daten an die Auswerteeinrichtung,
- Untersuchen der Daten auf das Erfülltsein der zu der ausgegebenen Sollvorgabe angegebenen Bedingung durch die Auswerteeinrichtung,
- Ausgeben einer Rückmeldung durch die Ausgabeeinrichtung zur Wahrnehmung durch die Person in Abhängigkeit von einem Ergebnis des Untersuchens.

Das Verfahren beinhaltet durch das Ausgeben der Sollvorgabe mittels der Ausgabeeinrichtung, dass die Person weiß, welche Töne (und wie) sie sie zu spielen hat. Es erfolgt dann eine Rückmeldung ("Feedback"), sodass die Person das Spiel und damit auch das Training optimieren kann, um möglichst ihre Muskulatur und ihr Bindegewebe in Mundhöhle und Rachen so zu trainieren, dass Atemwegs- und Schlafstörungen langfristig vermieden werden.

Vorzugsweise geht dem Schritt des Ausgebens einer Sollvorgabe voraus:
a) Empfangen einer Eingabe der Person zur Auswahl eines Trainingsprogramm und/oder
b) Erfassen des Gegenstands und/oder des Zeitpunkts, zu zumindest einer früheren Sollvorgabe, und/oder
c) Erfassen des Gegenstands und/oder des Zeitpunkts zu zumindest einer früheren Rückmeldung,
wobei die ausgegebene Sollvorgabe von zumindest einem aus der Eingabe, dem zumindest einem erfassten Gegenstand einer früheren Sollvorgabe, dem zumindest einem erfassten Zeitpunkt einer früheren Sollvorgabe, dem zumindest einem erfassten Gegenstand einer früheren Rückmeldung und dem zumindest einem erfassten Zeitpunkt einer früheren Rückmeldung abhängig ausgegeben wird. Somit kann sich die Person ein bestimmtes Trainingsprogramm aussuchen, oder aber zusätzlich oder alternativ ist das Trainingsprogramm von dem bisher durchlaufenen Training und/oder dem bisherigen Trainingserfolg abhängig. Alternativ hierzu kann die Sollvorgabe von einem absoluten Zeitpunkt abhängig sein, etwa vom Datum her definiert sein. Es wird dann einfach ein durchgängiges Trainingsprogramm angeboten, unabhängig davon, ob die Person tatsächlich trainiert (und wie oft), oder nicht. Hat die Person beispielsweise drei Tage pausiert, sind möglicherweise inzwischen die Trainingsansprüche gestiegen, was dann das Training nach der Pause erst recht anspruchsvoller macht.

Vorzugsweise ändert sich die zu einer Sollvorgabe angegebene Bedingung in Abhängigkeit von zumindest einem aus Gegenstand und Zeitpunkt einer früheren Sollvorgabe und Gegenstand und Zeitpunkt einer früheren Rückmeldung. So kann das Training flexibler erfolgen. Trifft die das Blasinstrument spielende Person Tonhöhe und/oder Lautstärke nicht ganz so wie gewünscht, kann das Trainingsprogramm etwas großzügigere Maßstäbe anlegen. Trifft die Person Tonhöhe und Lautstärke besonders gut, können umgekehrt die Anforderungen nach und nach auch gesteigert werden. Alternativ hierzu ist vorab ein durchgehendes Trainingsprogramm festgelegt. Bei der Festlegung können aber die Ergebnisse von Messungen an der Person (etwa Bildgebung in Mundhöhle und Rachen) und/ oder mit Beteiligung der Person ("Probeblasen") einbezogen werden.

Weitere Einzelheiten der Erfindung und insbesondere beispielhafte Ausführungsformen der erfindungsgemässen Vorrichtung werden im Folgenden anhand der beigefügten Zeichnungen erläutert. Es zeigen:
- Fig. 1: eine Explosionsdarstellung eines Blasinstrumentes, wie es bei der erfindungsgemäßen Vorrichtung einsetzbar ist,
- Fig. 1b: eine Explosionsdarstellung des Blasinstrumentes aus Fig. 1a ohne seinen oberen Deckel,
- Fig. 1c: den Blaskörper des Blasinstruments aus Fig. 1b in perspektivischer Ansicht,
- Fig. 2: einen Schnitt durch den Blaskörper aus Fig. 1c,
- Fig. 3: einen seitlichen Schnitt durch das in Fig. 1a gezeigte Blasinstrument im zusammengebauten Zustand,
- Fig. 4: eine alternative Ausführungsform von Deckel und Unterboden des Blasinstruments, das in Fig. 1a gezeigt ist,
- Fig. 5: das Blasinstrument aus Fig. 1a mit zusätzlichen Einrichtungen, wie sie bei einer ersten Ausführungsform der erfindungsgemäßen Vorrichtung genutzt werden,
- Fig. 6: das Blasinstrument aus Fig. 1a mit zusätzlichen Einrichtungen, wie sie bei einer zweiten Ausführungsform der erfindungsgemäßen Vorrichtung genutzt werden,
- Fig. 7: das Blasinstrument aus Fig. 1a mit zusätzlichen Einrichtungen, wie sie bei einer dritten Ausführungsform der erfindungsgemäßen Vorrichtung genutzt werden,
- Fig. 8: die vollständige Vorrichtung gemäß einer Ausführungsform der Erfindung unter Einsatz eines Smartphones, und
- Fig. 9: die vollständige Vorrichtung gemäß einer weiteren Ausführungsform der Erfindung unter Einsatz eines Personalcomputers,

Fig. 10 ein Flussschaubild zur Veranschaulichung eines nicht beanspruchten Verfahrens.

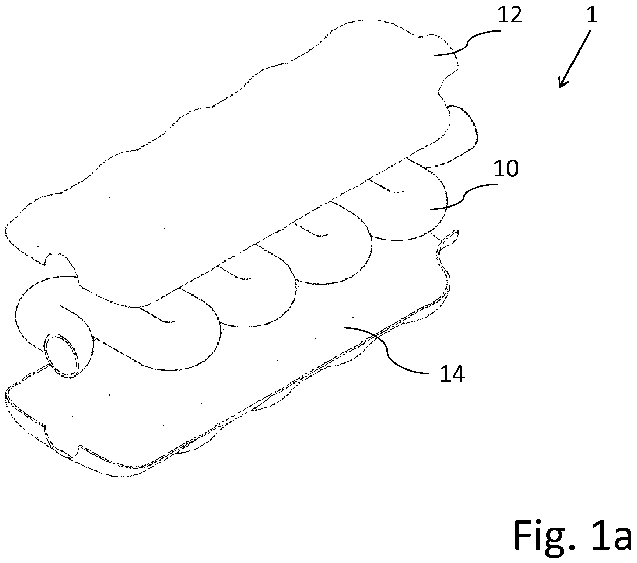

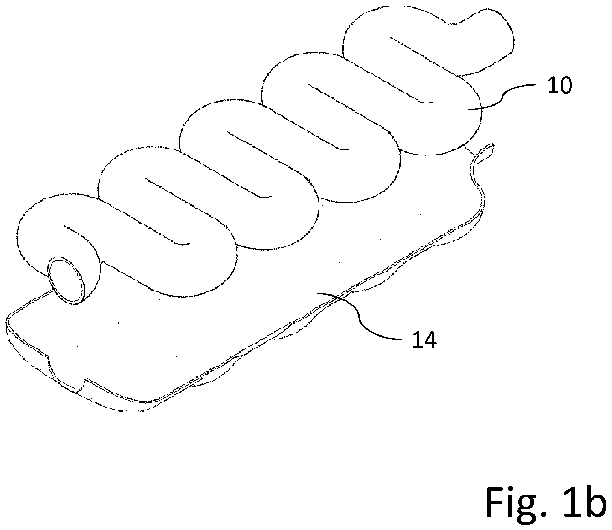

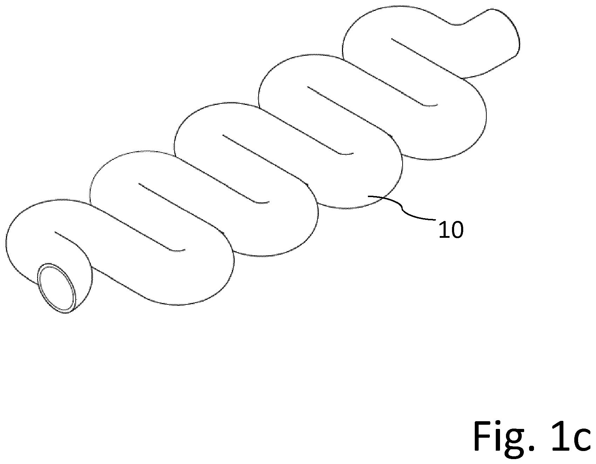

Das in Fig. 1a in Explosionsdarstellung gezeigte, im Ganzen mit 1 bezeichnete Blasinstrument umfasst den eigentlichen Blaskörper 10, einen oberen Deckel 12 und einen Unterboden 14. Die Figuren 1b und 1c zeigen jeweils nur Teile des Blasinstruments zur besseren Sichtbarmachung derselben.

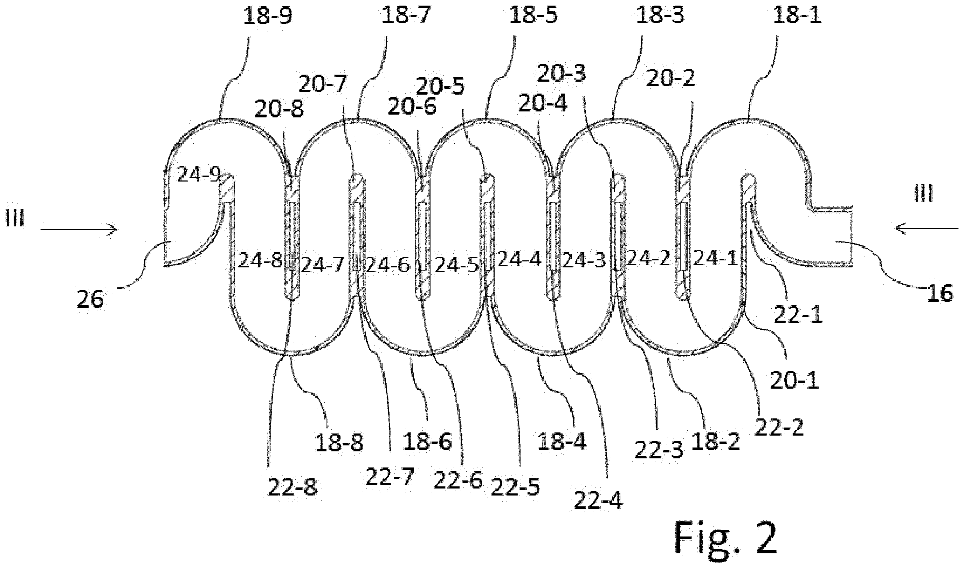

Der Blaskörper 10 ist im Schnitt in Fig. 2 dargestellt. Er umfasst einen Einblasstutzen 16, auf den in Richtung des Eintretens von Luft beim Blasen eine erste Windung 18-1 folgt, eine zweite Windung 18-2 folgt, und weitere Windungen 18-3, 18-4, 18-5, 18-6, 18-7, 18-8 und 18-9 folgen. Zwischen den beiden Windungen 18-1 und 18-3 ist ein geradliniger Wandabschnitt 20-1 angeordnet, zwischen den Windungen 18-2 und 18-3 ein geradliniger Wandabschnitt 20-2, und so stehen paarweise zwischen Windungen jeweils geradlinige Wandabschnitte 20-3, 20-4, 20-5, 20-6 und 20-7. Zwischen dem Einlassstutzen 16 und dem geradlinigen Wandabschnitt 20-1 befindet sich ein offener Raum 22-1. Im Bereich der geradlinigen Wandabschnitte 20-2, 20-3, 20-4 etc. befinden sich Hohlräume 22-2, 22-3, 22-4, 22-5, 22-6, 22-7, 22-8. Die Hohlräume können, müssen aber nicht, so ausgebildet sein, dass sie als Resonanzräume wirken und zur Verstärkung des Schalls beitragen. Zwischen den beiden geradlinigen Wandabschnitten 20-1 und 20-2 befindet sich ein rohrförmiger Abschnitt 24-1, zwischen den geradlinigen Wandabschnitten 20-2 und 20-3 der Rohrabschnitt 24-2, etc. Der letzte Rohrabschnitt 24-8 geht in einen Rohrabschnitt 24-9 über, der schließlich in den Austrittstutzen 26 mündet.

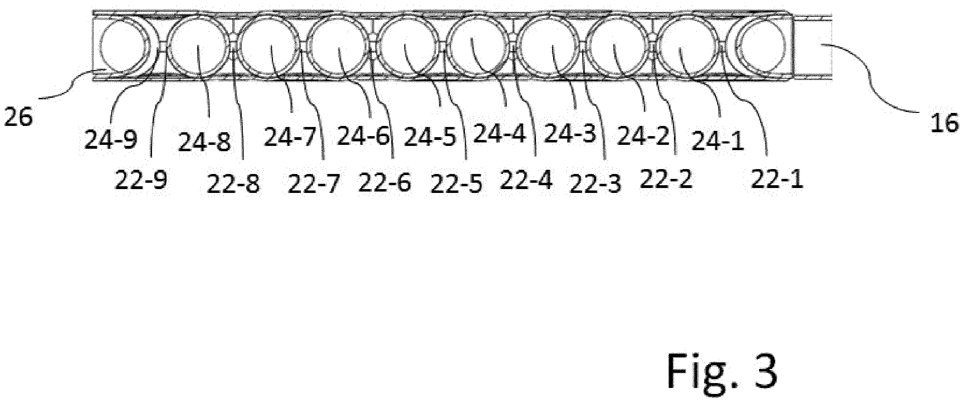

Die Fig. 3 zeigt im Schnitt III-III aus Fig. 2 die jeweiligen Rohrabschnitte 24-1, 24,2, 24-3 etc. mit den dazwischen befindlichen Kammern 22-3, 22-4, etc. Die Rohrabschnitte sind von kreisförmigem Durchschnitt und haben einen Durchmesser von zwischen 1 und 5 cm, beispielsweise von zwischen 2,2 und 2,8 cm.

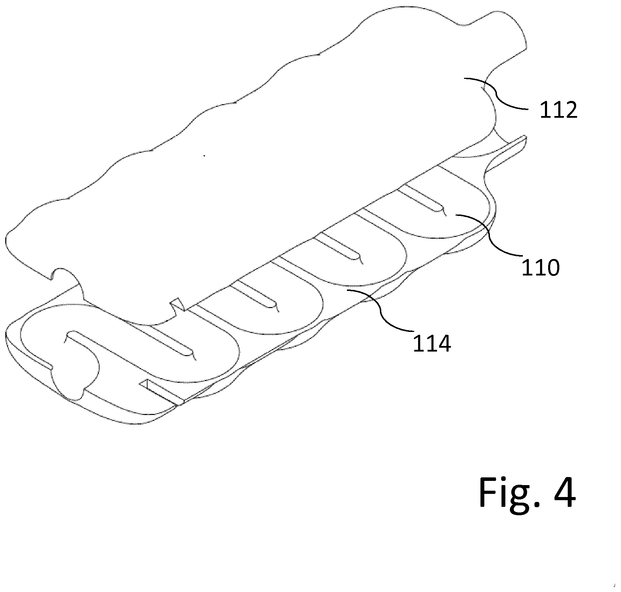

Bei der alternativen Ausführungsform des Blasinstruments gemäß Fig. 4 sind nur zwei Teile vorgesehen, nämlich der obere Deckel 112 und der Unterboden 114, wobei das Blasinstrument 101 durch Hohlräume 110 im Unterboden 114 bereitgestellt ist, die durch entsprechende (in der Figur nicht gezeigte) Hohlräume im oberen Deckel zur Bildung des Durchtrittsraums für Luft im Blasinstrument 101 ergänzt sind.

Zur erfindungsgemäßen Vorrichtung gehört eine Schallaufnahmeeinrichtung, mithilfe derer der Schall erfasst wird. Dies wird anhand von Abwandlungen des Blasinstruments 1 aus Fig. 1a erläutert, wobei die hier beschriebenen Ausführungsformen auch entsprechend angepasst bei der Alternative 101 gemäß Fig. 4 einsetzbar sind.

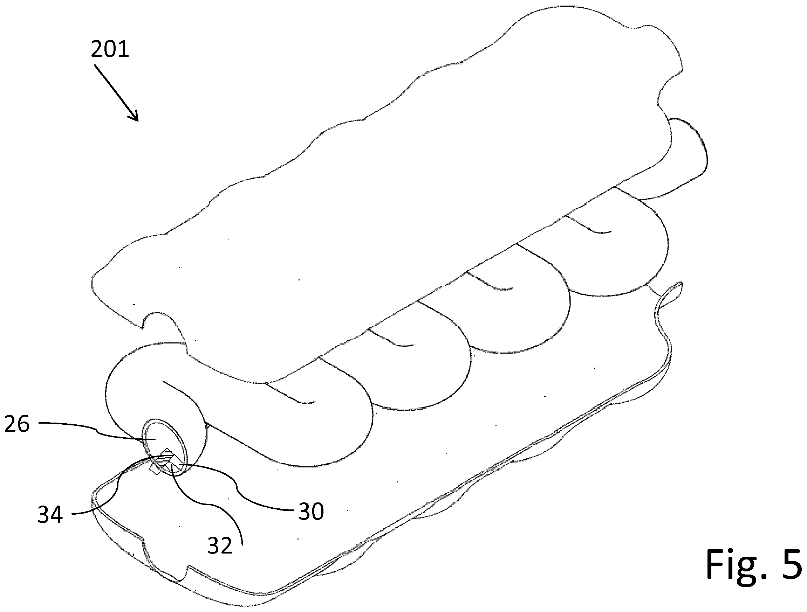

Bei einer ersten Variante des Blasinstruments mit eingebauter Schallaufnahmeeinrichtung, wie sie in Fig. 5 gezeigt und dort mit 201 bezeichnet ist, ist im Bereich des Austrittsstutzens 26 für Luft ein Mikrofon 30 vorgesehen, dem eine Sendeeinrichtung 32 zugeordnet ist, wobei Mikrofon 30 und Sendeeinrichtung 32 über einen Energiespeicher 34 (beispielsweise in Form eines Lithium-Ionen-Akkus) mit Energie versorgt werden. Der Energiespeicher 34 kann entweder austauschbar sein oder durch Anschluss eines nicht gezeigten Ladekabels mit einer externen Stromquelle wie einer Steckdose gekoppelt werden, um aufgeladen zu werden.

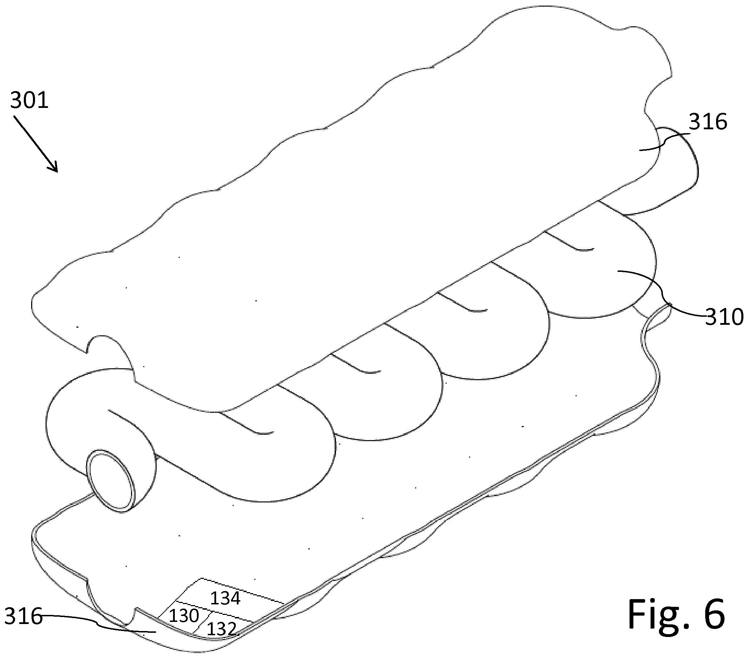

Bei der Variante gemäß Fig. 6, die im ganzen mit 301 bezeichnet ist, ist ein Mikrofon 130 in einem Hohlraum im Unterboden 314 außerhalb des Blaskörpers 310 vorgesehen und mit einer Wandung 316 des Unterbodens in der Nähe des Austrittsstutzens 326 gekoppelt, um darin erzeugten Schall aufzunehmen. Die Sendeeinrichtung 132 und ein Energiespeicher 134 sind ebenfalls in diesem Bereich vorgesehen.

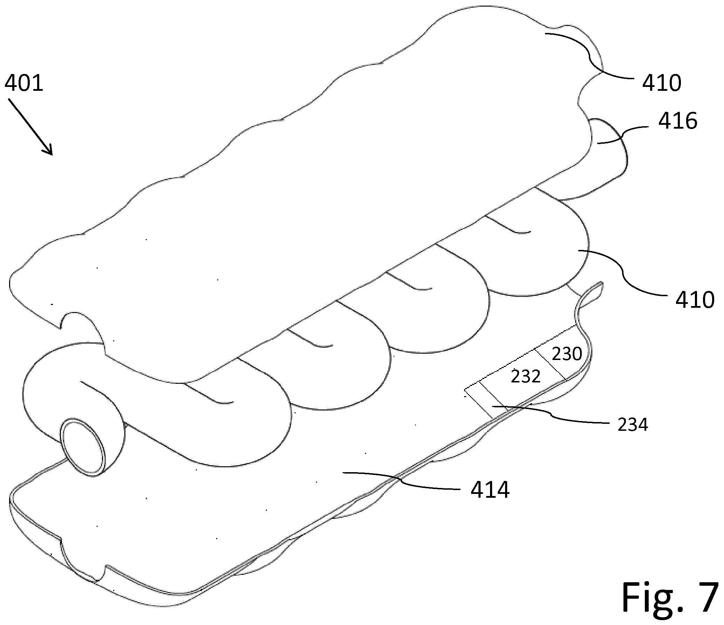

Bei einer weiteren, in Fig. 7 gezeigten und dort mit 401 bezeichneten Variante sind ein Mikrofon 230, eine Sendeeinrichtung 232 und ein Energiespeicher 234 ebenfalls in einem Hohlraum des Unterbodens 414 bereitgestellt, außerhalb des Blaskörpers 410, wobei hier sich das Mikrofon 230 in der Nähe des Einblasstutzens 416 befindet.

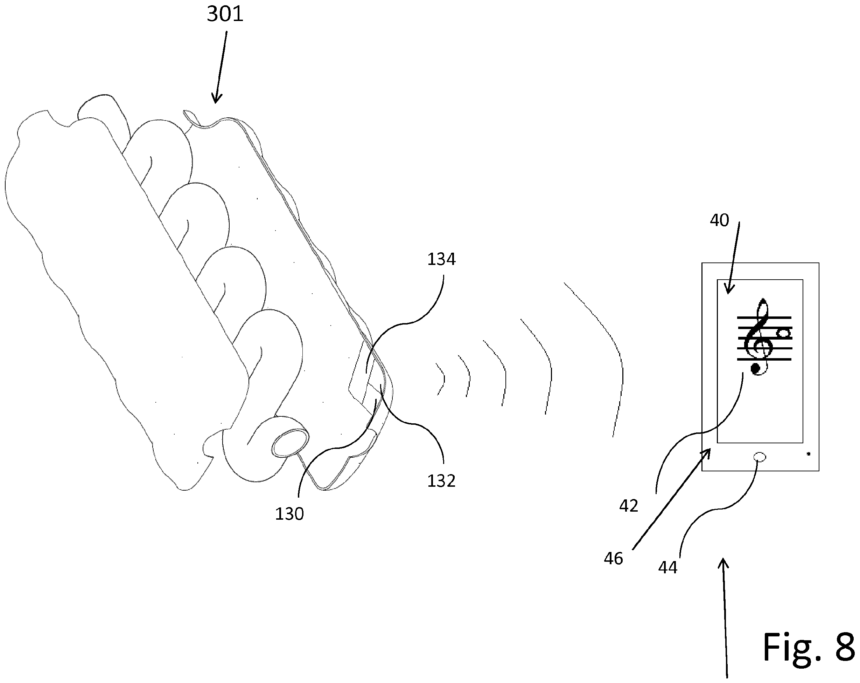

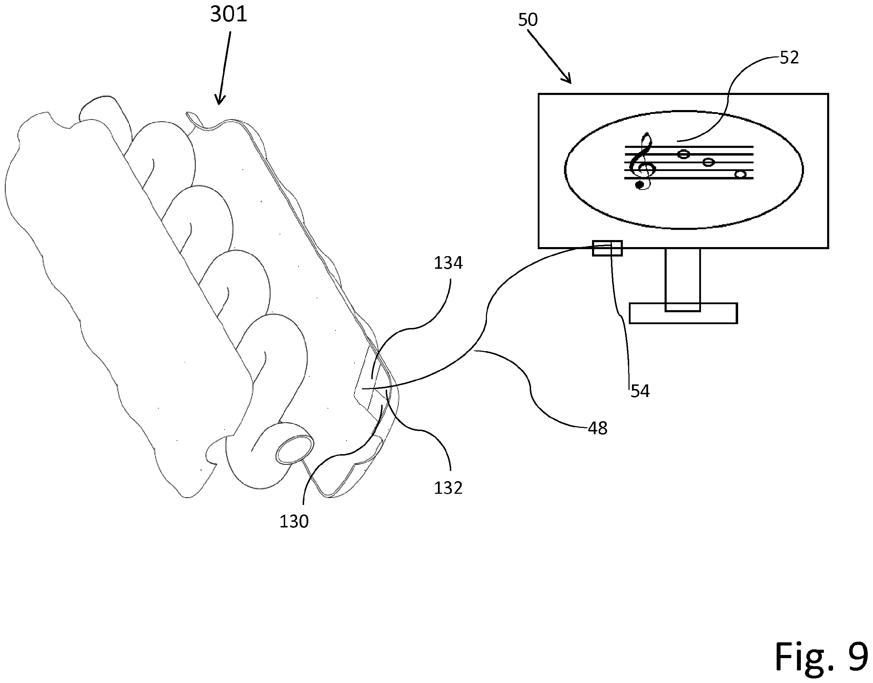

Die Umsetzung der Erfindung wird anhand der Figuren 8 und 9 erläutert, die an die Ausführungsform 301 des Blasinstruments gemäß Fig. 6 anknüpfend gezeigt, gleichermaßen aber auch bei den anderen Ausführungsformen gemäß Fig. 5 oder 7 umsetzbar ist. Beim Blasen des Blasinstruments erfasst das Mikrofon 130 den so erzeugten Schall, und die Sendeeinrichtung 132 sendet die erfassten Schalldaten drahtlos, beispielsweise unter Einsatz eines Bluetooth®-Standards, an eine entsprechende Empfangseinrichtung 46 eines Smartphones 40. Das Smartphone 40 umfasst ein Display 42. Beispielhaft dargestellt ist auf dem Display 42 eine Note, um zu symbolisieren, dass der das Blasinstrument spielenden Person (also der Person, die ihre Muskulatur und das Bindegewebe in Mundhöhle und Rachen trainiert) eine Vorgabe gemacht wird, welchen Ton sie zu spielen hat. Eingaben können zudem über eine Eingabevorrichtung 44, wie etwa einen Schalter, Touchscreen oder dergleichen am Smartphone 40 gemacht werden.

Bei der alternativen Ausführungsform gemäß Fig. 9 ist die Sendeeinrichtung 132 über ein Kabel 48 mit einem Personalcomputer 50 gekoppelt, der einen Bildschirm 52 und eine Schnittstelle 54 für das Kabel aufweist.

Ein nicht beanspruchtes Verfahren beginnt in Schritt S10 ("Start") mit dem Aktivieren der elektronischen Teile, beispielsweise dem Einschalten des Blasinstruments mit einem in den Figuren nicht gezeigten Schalter und dem Einschalten des Smartphones 40 oder des Personalcomputers 50. In Schritt S12 erfolgt durch den Trainierenden die Auswahl eines Programms, nämlich eines Trainingsprogramms. Alternativ aktiviert er einfach ein einziges Trainingsprogramm, etwa durch Einschalten einer App. Es handelt sich hierbei um ein Trainingsprogramm, das dem Benutzer Vorgaben macht, wie er das Blasinstrument zu spielen hat, um die Muskulatur und das Bindegewebe in Mundhöhle und Rachenoptimal zu trainieren, damit Atemwegs- und Schlafstörungen langfristig vermieden werden. Hierbei kommt es darauf an, dass ein bestimmtes Trainingsprogramm möglichst genau durchlaufen wird. Jedes Programm umfasst eine Anzahl n an Sollvorgaben. Es wird mit der ersten begonnen (i:= 1 wird in Schritt S14 gesetzt). In Schritt S16 wird über das Display 42 des Smartphones oder den Bildschirm 52 des Personalcomputers die Sollvorgabe, Teil i ausgegeben. Der Trainierende betätigt nun durch Blasen in den Einblasstutzen 416 das Blasinstrument, und mithilfe des Mikrofons 132, das im Beispielsfalle der Figuren Teil des Blasinstruments 301 ist, werden die Schalldaten im Schritt S18 empfangen. Eine Auswerteeinrichtung (ein Datenverarbeitungsprozessor oder dergleichen im Smartphone 40 oder im Personalcomputer 50) vergleicht auf das Erfülltsein der Sollvorgabe nach einem vorbestimmten Kriterium. Hierfür kann in einem nicht gezeigten Datenspeicher zu jeder Sollvorgabe ein Bereich angegeben sein, der das Erfülltsein der Sollvorgabe definiert. Beispielsweise kann die Sollvorgabe beinhalten, dass eine bestimmte Tonhöhe zu spielen ist, dann können benachbarte Tonhöhen noch als akzeptabel gelten. So kann in einem Notensystem der zu spielende Ton angezeigt werden (etwa in Grün) und der tatsächlich gespielte Ton kann zusätzlich angezeigt werden (etwa in Rot). Ist beispielsweise der Ton zu tief gespielt, wird mit roter Farbe im Notensystem der gespielte Ton angezeigt. Erhöht dann der Spieler den Ton, wandert die Note in der Darstellung nach oben und wird, sobald sie in das definierte Tonintervall fällt, grün.

Beispielweise kann ferner eine bestimmte Lautstärke vorgegeben sein, die erzielt werden kann, dann können auch verwandte Lautstärken erzielt werden. Auch das Umsetzen einer bestimmten Lautstärke kann mittels laufender Rückmeldung erfolgen: typischerweise wird man hier eine Balkendarstellung wählen, bei der die Länge des Balken einer Lautstärke entspricht; je länger der Balken, desto lauter ist die Lautstärke. Zu der zu erzielenden Lautstärke wird ein grüner Strich angezeigt. Der Balken bleibt solange rot, bis das Intervall erreicht ist, in dem die Lautstärke liegen soll, und dann wird der Balken grün gefärbt. Andere Arten der Darstellung sind selbstverständlich möglich. Das Erfülltsein der Sollvorgabe ist durch Kombination von Tonhöhe und Lautstärke definiert, die beispielsweise als 2-Tupel angegeben werden könnten. (Es kann Lautstärken geben, die bei einer bestimmten Tonhöhe akzeptiert werden, bei einer anderen Tonhöhe aber nicht).

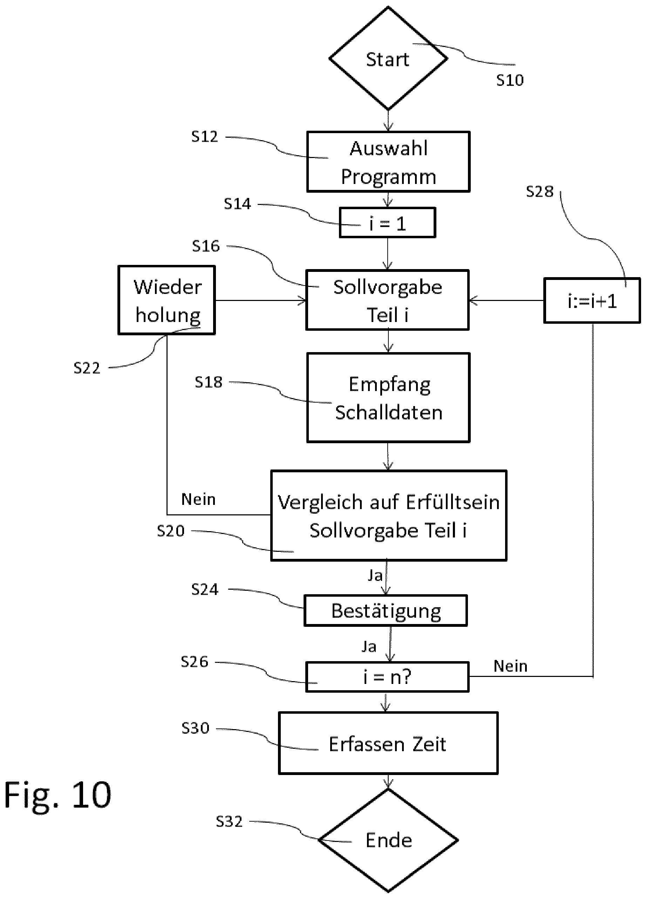

In Schritt S20 wird geprüft, ob die Sollvorgabe, Teil i erfüllt ist. Ist dies nicht der Fall, erfolgt eine Wiederholung gemäß Schritt S22 und die Rückkehr zum Schritt S10. Hierbei kann gegebenenfalls die Anzahl der Wiederholungen gezählt werden, um ein Ausstiegsszenario zu ermöglichen, was in der Fig. 10 allerdings nicht gezeigt ist. Falls in Schritt S20 hingegen die Sollvorgabe erfüllt ist, wird in Schritt S24 eine Ausgabe an den Trainierenden gegeben, um den Erfolg anzuzeigen. Ferner wird geprüft, ob es sich um die letzte Sollvorgabe (i = n) handelte. Diese Prüfung erfolgt in Schritt S26. Ist das Ereignis negativ, wird die Variable i um 1 erhöht, Schritt S28 beinhaltet i:=(i+1); und es wird zu Schritt S16 zurückgekehrt, die nächste Sollvorgabe angezeigt, abermals werden Schalldaten empfangen und es wird nochmals auf das Erfülltsein geprüft. Ist schließlich i = n erreicht, dann wird in Schritt S30 die Zeit des Erreichens erfasst (Datum und Uhrzeit); gegebenenfalls können zusätzliche Informationen erfasst werden, wie etwa Anzahl der Wiederholungen gemäß Schritt S22 oder die Gesamtdauer, die der Trainierende benötigte, um alle Sollvorgaben zu durchlaufen. Mit dieser Information kann dem Benutzer nach dem nächsten Start eine geeignete Vorgabe zur Auswahl von Programmen gegeben werden. Schließlich werden alle elektrischen Geräte ausgeschaltet, und das Verfahren endet in Schritt S32.

Das Verfahren kann auch ohne eine vorherige Festlegung der Anzahl der Sollvorgaben auskommen, d.h. n wird so hoch gesetzt, dass es nie erreicht wird. Der Trainierende kann dann selbsttätig das Training beenden.

Insgesamt ist das anhand der Fig. 10 beschriebene Verfahren nur beispielhaft, und bei der Umsetzung können manche Schritte und Aspekte entfallen oder andere hinzuergänzt werden.

## Patentansprüche

1. Vorrichtung zum Trainieren der Muskulatur und des Bindegewebes in Mundhöhle und Rachen einer Person, umfassend ein Blasinstrument (201,301,401) und eine Einrichtung (30,130,230) zum Erfassen eines von der Person mit dem Blasinstrument (201,301,401) erzeugten Schalles, eine Einrichtung (40,50) zum Auswerten des erfassten Schalles und eine Einrichtung (42,52) zum Ausgeben einer in Abhängigkeit von einem Ergebnis des Auswertens definierten Rückmeldung an die das Blasinstrument (201,301,401) spielende Person,
wobei die Einrichtung zum Erfassen ein in dem Blasinstrument angeordnetes Mikrofon (30,130,230) und/oder einen in dem Blasinstrument angeordneten piezoelektrischen Sensor umfasst,
**dadurch gekennzeichnet, dass** das Blasinstrument zwischen fünf und zwölf Windungen in Folge hintereinander aufweist, und
wobei die Einrichtung zum Auswerten einen Datenspeicher aufweist, in welchem zumindest eine Datei mit Information gespeichert ist, welche für das Spielen des Blasinstruments (201,301,401) vorgesehene Sollvorgaben für die Kombination von Tonhöhe und Lautstärke angibt (S16).

2. Vorrichtung nach Anspruch 1, bei der die Einrichtung (40,50) zum Auswerten mit der Einrichtung (42,52) zum Ausgeben gekoppelt ist und die Einrichtung (42,52) zum Ausgeben auch zum Ausgeben einer Meldung an die das Blasinstrument (201,301,401) spielende Person ausgelegt ist, mit der der Person eine Sollvorgabe angegeben wird.

3. Vorrichtung nach Anspruch 1 oder 2, bei der die Information in der zumindest einen Datei zu zumindest einem Teil der Sollvorgaben jeweils auch angibt, welche Bedingung durch von der Einrichtung (30,130,230) zum Erfassen des Schalles an die Einrichtung (40,50) zum Auswerten weitergeleitete Daten erfüllt sein muss, damit diese Sollvorgabe erfüllt ist, und bei der die Einrichtung (40,50) zum Auswerten dazu ausgelegt ist, auf das Erfülltsein der Bedingung zu prüfen.

4. Vorrichtung nach einem der vorhergehenden Ansprüche, bei der die Einrichtung zum Erfassen eine mit dem Mikrofon (30,130,230) beziehungsweise dem piezoelektrischen Sensor gekoppelte und in oder an dem Blasinstrument angeordnete Sendeeinrichtung (32,132,232) zum Übermitteln von mithilfe des Mikrofons beziehungsweise des piezoelektrischen Sensors erfassten Daten an die Einrichtung (40,50) zum Auswerten aufweist.

5. Vorrichtung nach Anspruch 4, bei der die Einrichtung (40,50) zum Auswerten ausserhalb des Blasinstruments bereitgestellt ist.

6. Vorrichtung nach Anspruch 5, bei der die Sendeeinrichtung ausgelegt ist, die mithilfe des Mikrofons beziehungsweise des piezoelektrischen Sensors erfassten Daten drahtlos an die Einrichtung (40,50) zum Auswerten zu senden.

7. Vorrichtung nach einem der vorhergehenden Ansprüche, bei der das Blasinstrument (201,301,401) einen Speicher (34,134,234) für elektrische Energie aufweist, um erstens das Mikrofon (30,130,230) beziehungsweise den piezoelektrischen Sensor und/oder zweitens die Sendeeinrichtung (32,132,232) zu betreiben.

8. Vorrichtung nach einem der vorhergehenden Ansprüche, bei der die Einrichtung zum Auswerten des erfassten Schalles und eine Einrichtung (42,52) zum Ausgeben einer in Abhängigkeit vom Ergebnis des Auswertens definierten Rückmeldung an die das Blasinstrument spielende Person durch ein mobiles Datenverarbeitungsgerät (40), insbesondere ein Smartphone (40) oder einen Tabletcomputer, bereitgestellt ist, auf dem ein entsprechendes Programm, insbesondere eine App, gespeichert ist.

9. Vorrichtung nach einem der vorhergehenden Ansprüche, bei der das Blasinstrument (201,301,401) zumindest abschnittsweise aus Holz oder zumindest abschnittsweise aus Kunststoff besteht.

## Claims

1. An apparatus for training the muscles and the connective tissue in the oral cavity and throat of a person, comprising a wind instrument (201, 301, 401) and
a device (30, 130, 230) for detecting a sound produced by a person by means of the wind instrument (201, 301, 401), a device (40, 50) for evaluating the detected sound and a device (42, 52) for outputting a response defined as a function of a result of the evaluation to the person playing the wind instrument (201, 301, 401),
wherein the device for detecting comprises a microphone (30, 130, 230) arranged in the wind instrument and/or a piezoelectric sensor arranged in the wind instrument,
**characterized in that**
the wind instrument has between five and twelve bends consecutively in succession,
wherein
the device for evaluating comprises a data storage device in which at least one file with information is stored which gives target specifications (S16) provided for the combination of pitch and loudness for the playing of the wind instrument (201, 301, 401) .

2. The apparatus according to claim 1, in which the device (40, 50) for evaluating is coupled to the device (42, 52) for outputting and the device (42, 52) for outputting is designed to output a message to the person playing the wind instrument (201, 301, 401) by means of which a target specification is given to the person.

3. The apparatus according to claim 1 or 2, in which the information in the at least one file relating to at least a portion of the target specifications in each case also specifies which condition must be satisfied by the data relayed from the device (30, 130, 230) for detecting the sound to the device (40, 50) for evaluating so that this target specification is satisfied and in which the device (40, 50) for evaluating is designed to check that the condition is satisfied.

4. The apparatus according to one of the preceding claims
in which the device for detecting comprises a transmitting device (32, 132, 232) coupled to the microphone (30, 130, 230) or the piezoelectric sensor and arranged in or on the wind instrument for transmitting data recorded with the aid of the microphone or the piezoelectric sensor to the device (40, 50) for evaluating.

5. The apparatus according to claim 4, in which the device (40, 50) for evaluating is provided outside the wind instrument.

6. The apparatus according to claim 5, in which the transmitting device is designed to transmit the data recorded by means of the microphone or the piezoelectric sensor wirelessly to the device (40, 50) for evaluating.

7. The apparatus according to one of the preceding claims, in which the wind instrument (201, 301, 401) has a storage device (34, 134, 234) for electrical energy in order to firstly operate the microphone (30, 130, 230) or the piezoelectric sensor and/or secondly operate the transmitting device (32, 132, 232).

8. The apparatus according to one of the preceding claims in which the device for evaluating the recorded sound and a device (42, 52) for outputting a response defined as a function of the result of the evaluation to the person playing the wind instrument is provided by a mobile data processing device (40), in particular a smartphone (40) or a tablet computer, on which a corresponding program, in particular an app is stored.

9. The apparatus according to one of the preceding claims in which the wind instrument (201, 301, 401) consists of wood at least in sections or of plastic at least in sections.

## Revendications

1. Dispositif destiné à exercer la musculature et le tissu conjonctif dans la cavité buccale et le pharynx d'une personne comprenant
un instrument à vent (201, 301, 401) et un système (30, 130, 230) pour saisir un son produit par la personne avec l'instrument à vent (201, 301, 401), un système (40, 50) pour évaluer le son produit et un système (42, 52) pour délivrer à la personne jouant de l'instrument à vent (201, 301, 401) une réponse rétroactive définie en fonction d'un résultat de l'évaluation,
sachant que le système pour saisir comprend un microphone (30, 130, 230) disposé dans l'instrument à vent et/ou un capteur piézoélectrique disposé dans l'instrument à vent,
**caractérisé en ce que**
l'instrument à vent comporte entre cinq et douze enroulements à la suite l'une de l'autre, et
sachant que le système pour évaluer comporte une mémoire de données dans laquelle est mémorisé au moins un fichier avec une information, laquelle indique (S16) des consignes théoriques prévues pour la combinaison de hauteur de ton et de volume sonore pour jouer de l'instrument à vent (201, 301, 401).

2. Dispositif selon la revendication 1 pour lequel le système (40, 50) pour évaluer est couplé au système (42, 52) pour émettre et le système (42, 52) pour émettre est conçu également pour émettre un message à la personne jouant de l'instrument à vent (201, 301, 401) avec lequel une consigne théorique est indiquée à la personne.

3. Dispositif selon la revendication 1 ou 2 pour lequel l'information dans au moins un fichier pour au moins une partie des consignes théoriques indique respectivement également quelle condition doit être remplie par les données transmises par le système (30, 130, 230) pour saisir le son sur le système (40, 50) pour évaluer afin que cette consigne théorique soit satisfaite et pour lequel le système (40, 50) pour évaluer est conçu pour vérifier qu'il est bien satisfait à cette condition.

4. Dispositif selon l'une quelconque des revendications précédentes pour lequel le système pour saisir comporte un système d'émission (32, 132, 232) couplé au microphone (30, 130, 230) ou bien au capteur piézoélectrique et disposé dans ou sur l'instrument à vent pour transmettre des données saisies à l'aide du microphone ou bien du capteur piézoélectrique au système (40, 50) pour évaluaer.

5. Dispositif selon la revendication 4 pour lequel le système (40, 50) est préparé pour évaluer en dehors de l'instrument à vent.

6. Dispositif selon la revendication 5 pour lequel le système d'émission est conçu pour envoyer sans fil au système (40, 50) pour évaluer les données saisies à l'aide du microphone ou bien du capteur piézoélectrique.

7. Dispositif selon l'une quelconque des revendications précédentes pour lequel l'instrument à vent (201, 301, 401) comporte un accumulateur (34, 134, 234) pour l'énergie électrique pour faire fonctionner, premièrement le microphone (30, 130, 230) ou bien le capteur piézoélectrique et/ou deuxièmement le système d'émission (32, 132, 232).

8. Dispositif selon l'une quelconque des revendications précédentes pour lequel sont préparés le système pour évaluer le son saisi et un système (42, 52) pour délivrer une réponse rétroactive définie en fonction du résultat de l'évaluation à la personne jouant de l'instrument à vent par un appareil de traitement des données mobile (40), en particulier un téléphone intelligent (40) ou une tablette, sur lequel est mémorisé un programme correspondant, en particulier une application.

9. Dispositif selon l'une quelconque des revendications précédentes pour lequel l'instrument à vent (201, 301, 401) est composé au moins en partie de bois ou au moins en partie de matière plastique.
